(19) Europäisches Patentamt
European Patent Office
Office européen des brevets

(11) Numéro de publication: 0 084 744 B1

(12) FASCICULE DE BREVET EUROPEEN

(45) Date de publication du fascicule du brevet: 21.11.84

(51) Int. Cl.³: C 07 C 147/06

(21) Numéro de dépôt: 82401217.3

(22) Date de dépôt: 30.06.82

(54) **Procédé de sulfonylation d'halogénobenzènes.**

(30) Priorité: 21.01.82 FR 8200879

(43) Date de publication de la demande: 03.08.83 Bulletin 83/31

(45) Mention de la délivrance du brevet: 21.11.84 Bulletin 84/47

(84) Etats contractants désignés: AT BE CH DE FR GB IT LI LU NL SE

(56) Documents cités:
DE - A - 1 645 153
FR - A - 2 053 336

(73) Titulaire: RHONE-POULENC SPECIALITES CHIMIQUES, "Les Miroirs" 18, Avenue d'Alsace, F-92400 Courbevoie (FR)

(72) Inventeur: Desbois, Michel, 526, Chemin du Bois, F-69140-Rillieux (FR)

(74) Mandataire: Cazes, Jean-Marie et al, RHONE-POULENC RECHERCHES Service Brevets Chimie et Polymères 25, quai Paul Doumer, F-92408 Courbevoie Cedex (FR)

EP 0 084 744 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

**Description**

La présente invention a pour objet un procédé de sulfonylation d'halogénobenzènes.

L'invention concerne plus particulièrement la préparation de sulfons par réaction de sulfonylation c'est-à-dire par réaction de l'halogénobenzène corrspondant et d'un acide sulfonique, un précurseur ou un dérivé de cet acide.

On connait dans l'art antérieur (OLAH »Friedel-Crafts and Related Reactions — III — 1964 — Part II Interscience Publishers« p. 1319 et suivantes) des réactions de sulfonylation en présence de catalyseurs comme $AlCl_3$, $FeCl_3$, $SbCl_5$ en milieu solvant organique, le substrat pouvant être également le solvant.

Ces procédés présentent des inconvénients qui tiennent essentiellement à la nature du catalyseur. En effet, il est nécessaire d'utiliser une quantité importante de catalyseur: il faut utiliser plus d'une mole de catalyseur comme le chlorure d'aluminium par mole de substrat car le groupement sulfonyle du réactif ou de la sulfone formée complexe mole à mole le chlorure d'aluminium d'où la nécessité de mettre en oeuvre plus d'une mole de ce dernier.

Cette quantité importante d'$AlCl_3$ enraine la mise en oeuvre d'une grande quantité d'eau pour son élimination. De plus, sa récupération est impossible industriellement.

La présente invention concerne un procédé de sulfonylation d'halogénobenzènes caractérisé en ce que l'on fait réagir l'halogéno benzène avec un acide sulfonique, un précurseur ou un dérivé de cet acide en présence de trifluorure de bore en quantité telle que la pression absolue de trifluorure de bore dans l'enceinte réactionnelle soit supérieure à 1 bar et en présence d'acide fluorhydrique comme solvant.

Le procédé de l'invention est d'autant plus surprenant qu'il est précisé dans l'art antérieur (OLAH précité page 1338) que le trifluorure de bore est inactif comme catalyseur dans les réactions de sulfonylation.

On entend au sens de la présente invention par halogénobenzène d'une part, ces produits proprement dits et, d'autre part, leurs homologues correspondant à la présence d'un plusieurs radicaux substituant le noyau benzènique.

Plus particulièrement encore, l'invention concerne la réaction des composés de formule:

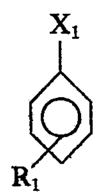

(I)

où:

- $X_1$ représente le chlore, le brome, l'iode ou le fluor
- et $R_1$ représente au moins un élément choisi parmi le groupe Comprenant l'hydrogène et les radicaux alkyle et alkoxy ayant de 1 à 6 atomes de carbone, les radicaux phényle et phénoxy substitués par au moins un groupement plus désactivant que le groupement $X_1$, OH, Cl, Br, I et F.

Les radicaux $R_1$ phényle et phénoxy doivent être substitués par des groupements plus désactivants que le groupement $X_1$ pour que la réaction de sulfonylation intervienne sur le novau benzénique porteur du groupement $X_1$. Dans le cas contraire, la réaction d'acylation se ferait sur le radical phényle ou phénoxy. On peut citer comme exemples de prougements plus désactvants que le groupements plus désactivants que le groupement $X_1$, les groupements $NO_2$, COOH, CN et cétones.

On peut citer comme exemples de composés (I): le chlorobenzène, le fluorobenzène, le bromobenzène, l'iodobenzène, l'o-, m- et p-fluorotoluène, l'o-, m- et p-dichlorobenzène, l'o-, m- et p-fluorophénol, l'o-, m- et p-fluorochlorobenzène, l'o-, m- et p-fluoroanisole, l'o-, m- et p-difluorobenzène, l'o-, m- et p-chlorotoluène, l'o-, m- et p-chloroanisole, le trifluorométhyl-4 chloro-4' biphényle et le trifluorométhyl-4 dichloro-2,4' diphényle oxyde.

On peut également citer les homologues chlorés, bromés et iodés des composés ci-dessus.

On entend au sens de la présente invention par acide sulfonique le précurseur ou le dérivé de cet acide, tous les réactifs de sulfonylation classiques décrits dans l'art antérieur.

Selon um mode de réalisation particulier de la présente invention l'acide sulfonique, le précurseur ou le dérive de cet acide répond à la formule générale:

$$R_2-SO_2X_2 \qquad (II)$$

dans laquelle:

$R_2$ représente un radical aliphatique ou aromatique

$X_2$ représente un halogène, OH, $OR_3$, $NH_2$, $NHR_4$, $NR_5R_6$, où $R_3$, $R_4$, $R_5$ et $R_6$ sont un radical aromatique ou aliphatique.

L'invention est particulièrement bien adaptée à la mise en oeuvre d'un composé de formule II dans laquelle $R_2$ représente un radical alkyle, phényle, alkylphényl, phénylalkyle ou phényle comportant au moins un subtituant comme par exemple un halogène, $NO_2$, CN, $NH_2$, COOH, $CF_3$, $CCl_3$, $CBr_3$.

On peut citer comme exemples de tels composés: clorure de paratoluènesulfonyle, chlorure de benzènesulfonyle, acide paratoluènesulfonique, acide benzènesulfonique, chlorure de méthanesulfonyle, chlorure de 2,4-diméthylbenzènesulfonyle, chlorure de métanitrobenzènesulfonyle, chlorure de méthoxy-2 benzènesulfonyle, chlorure de parachlorobenzènesulfonyle, fluorure de parahydroxybenzènesulfonyle, le chlorure de parabenzylbenzènesulfonyle.

Le procédé selon l'invention est mis en oeuvre de préférence en utilisant une quantité d'acide fluorhydrique telle que le rapport molaire de l'acide fluorhydrique à l'halogénobenzène est compris entre 5 et 50. Plus particulièrement, ce rapport est compris entre 10 et 30.

L'acide fluorhydrique mis en oeuvre est de préférence anhydre. La mise en oeuvre d'acide fluorhydrique aqueux entrainerait une consommation inutile de trifluorure de bore sous forme HF, $BF_3$, $H_2O$ ($H_3O^+BF_4^-$).

L'halogénobenzène et l'acide sulfonique, son précurseur ou son dérivé sont utilisés en quantité sensiblement équimolaire. Un léger excès du premier peut cependant être souhaitable.

On préfère plus particulièrement utiliser une quantité de trifluorure de bore telle que la pression absolue de $BF_3$ dans l'enceinte réactionnelle soit comprise entre 6 et 20 bars. Une pression supérieure à 20 bars n'est pas exclue de l'invention mais n'apporte pas d'avantages particuliers. Plus la pression sera élevée, plus la vitesse de réaction augmentera. L'homme de l'art adaptera donc la pression à l'économie du procédé.

Le procédé de l'invention est de préférence mis en oeuvre à une température comprise entre —20° C et 150° C.

Les temps de réaction sont généralement compris entre quelques minutes et plusieurs heures.

Les phénylsulfones obtenues selon le procédé de l'invention ont 5 pour formule générale:

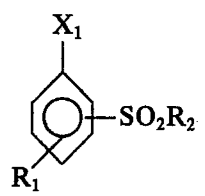

$X_1$, $R_1$ et $R_2$ ayant la signification précédente.

La position du groupement $-SO_2R_2$ par rapport auy groupements $X_1$ et $R_1$ obéit aux règles de substitution bien connues du chimiste organicien.

Les sulfones obtenues par le procédé de l'invention sont utiles comme intermédiaires de synthèse de composés ayant une activité pharmaceutique ou phytosanitaire.

On peut citer comme exemples de composès pouvant être préparés par le procédé selon l'invention: fluoro-4 méthyl-4' diphénylsulfone, fluoro-4 diphénylsulfone, fluoro-4 méthyl-3 diphénylsulfone, fluoro-4 diméthyl-3,4' diphénylsulfone, chloro-4 méthyl-4' diphénylsulfone,fluoro-4 phénylméthylsulfone, nitro-4 bromo-4' diphénylsulfone, fluoro-4 phényléthylsulfone, fluoro-4 méthyl-2 diphénylsulfone, fluoro-4 chloro-2 méthyl-4' diphénylsulfone, fluoro-5 hydroxy-2 chloro-4' diphénylsulfone, chloro-3 méthoxy-4 diphénylsulfone, chloro-2 dihydroxy-4,4' diphénylsulfone.

L'invention va être maintenant plus complètement décrite à l'aide des exemples que vont suivre, exemples que ne sauraient limiter l'invention.

## Exemple 1

Dans un réacteur inox de 250 ml muni d'un système d'agitation magnétique, on introduit aux environs de 0° C, 100 ml d'HF anhydre, 38,1 g (0,2 mole) de chlorure de p-toluène sulfonyle et 19,2 g (0,2 mole) de fluorobenzène. On ferme le réacteur puis on introduit du trifluorure de bore ($BF_3$) gazeux jusqu'à la pression constante de 10 bars. On laisse alors réagir sous agitation pendant 23 heures à la température ambiante. Après réaction, on décomprime le réacteur jusqu'à pression atmosphérique puis on coule le mélange réactionnel sur 200 g de glace pilée. On extrait alors le mélange hétérogène ainsi obtenu par trois fois 200 $cm^3$ de chlorure de méthylène. Les phases organiques sont lavées par trois fois 200 $cm^3$ d'eau, 1 fois 200 $cm^3$ d'une solution aqueuse à 3% de potasse et 2 fois 200 $cm^3$ d'au. La phase organique ainsi traitée est séchée sur sulfate de magnésium et le solvant est éliminé par distillation sous pression réduite. On recueille ainsi 49,5 g (rendement: 99%) de fluoro-4, méthyl-4' diphénylsulfone.

Exemple 2

On procédé comme à l'exemple 1 avec les composés et conditions suivantes:

| | |
|---|---|
| — Acide fluorhydrique anhydre | 100 g |
| — Fluorobenzène | 19,2 g 0,2 mole |
| — Chlorure de benzène sulfonyle | 35,3 g 0,2 mole |
| — Trifluorure de bore | 6 bars à 20° C |
| — Température | 40° C |
| — Durée | 1 heure |

On recueille 41 g (rendement: 87%) de fluoro-4, diphénylsulfone à 99,5% de pureté.

Exemple 3

On procède comme à l'exemple 1 avec les composés et conditions suivantes:

| | |
|---|---|
| — Acide fluorhydrique anhydre | 100 g |
| — o-fluorotoluène | 22 g 0,2 mole |
| — Chlorure de benzène sulfonyle | 35,5 g 0,2 mole |
| — Trifluorure de bore | 10 bars à 20° C |
| — Température | 20° C |
| — Durée | 4 heures |

On recueille 46 g (rendement: 92%) de fluoro-4 méthyl-3, diphénylsulfone à 97% de pureté.

Exemple 4

On procède comme à l'exemple 1 avec les composés et conditions suivantes:

| | |
|---|---|
| — Acide fluorhydrique anhydre | 100 g |
| — Fluorobenzène | 19,2 g 0,2 mole |
| — Acide p-toluène sulfonique | 34,4 g 0,2 mole |
| — Trifluorure de bore | 10 bars à 20° C |
| — Température | 80° C |
| — Durée | 6 heures |

On recueille 44 g (rendement: 88%) de fluoro-4 méthyl-4' diphénylsulfone.

Exemple 5

On procède comme à l'exemple 1 avec les composés et conditions suivantes:

| | |
|---|---|
| — Acide fluorhydrique anhydre | 100 g |
| — o-fluorotoluène | 22 g 0,2 mole |
| — Acide p-toluènesulfonique | 34,4 g 0,2 mole |
| — Trifluorure de bore | 10 bars à 20° C |
| — Température | 80° C |
| — Durée | 4 heures |

On recueille 43,9 g (rendement: 83%) de fluoro-4 diméthyl-3,4', diphénylsulfone à 80% de pureté.

Exemple 6

On procède comme à l'exemple 1 avec les composés et conditions suivantes:

| | |
|---|---|
| — Acide fluorhydrique anhydre | 100 g |
| — Bromobenzène | 31,4 g 0,2 mole |
| — Chlorure de benzènesulfonyle | 35,3 g 0,2 mole |
| — Trifluorure de bore | 6 bars à 20° C |

| | |
|---|---|
| — Température | 20°C |
| — Durée | 4 heures |

On recueille 51,6 g (rendement: 87%) de bromo-4 diphénylsulfone à 85% de pureté.

Exemple 7

On procède comme à l'exemple 1 avec les composés et conditions suivantes:

| | |
|---|---|
| — Acide fluorhydrique anhydre | 100 g |
| — Fluorobenzène | 19,2 g 0,2 mole |
| — Chlorure de méthanesulfonyle | 34,4 g 0,3 mole |
| — Trifluorure de bore | 10 bars à 20° C |
| — Température | 100° C |
| — Durée | 5 heures |

On recueille 16 g (rendement: 46%) de fluoro-4 phénylméthylsulfone à 70% de pureté.

Exemple 8

On procède comme à l'exemple 1 avec les composés et conditions suivantes:

| | |
|---|---|
| — Acide fluorhydrique anhydre | 100 g |
| — o-fluoroanisole | 25,8 g 0,2 mole |
| — Chlorure de p-toluène sulfonyle | 38,1 g 0,2 mole |
| — Trifluorure de bore | 10 bars à 20° C |
| — Durée | 1 heure |

On recueille 55 g (rendement: 97%) de fluoro-3 méthoxy-4, méthyl-4' diphénylsulfone à 92% de pureté.

Exemple 9

On procède comme à l'exemple 1 avec les composés et conditions suivantes:

| | |
|---|---|
| — Acide fluorhydrique anhydre | 100 g |
| — Fluoro-3 chlorobenzène | 13 g 0,1 mole |
| — Acide benzènesulfonique | 15,8 g 0,1 mole |
| — Trifluorure de bore | 15 bars à 20° C |
| — Température | 120° C |
| — Durée | 24 heures |

On recueille 19,8 g (rendement: 70%) d'un mélange de fluro-4, chloro-2 diphénylsulfone et de fluro-2, chloro-4 diphénylsulfone.

Exemple 10

On procède comme à l'exemple 1 avec les composés et conditions suivantes:

| | |
|---|---|
| — Acide fluorhydrique anhydre | 100 g |
| — Fluro-3 phénol | 11,2 g 0,1 mole |
| — Acide p-toluènesulfonique | 17,2 g 0,1 mole |
| — Trifluorure de bore | 10 bars à 20° C |
| — Température | 20° C |
| — Durée | 4 heures |

On recueille 25,3 g (rendement: 95%) d'un mélange de fluro-2 méthyl-4' hydroxy-4 diphénylsulfone et de fluro-4 méthyl-4' hydroxy-2 diphénylsulfone. Dans cet exemple, le lavage par la solution aqueuse de potasse à 3% a été supprimé afin de ne pas extraire le phénol formé.

## Revendications

1. Procédé de sulfonylation d'halogénobenzènes caractérisé en ce que l'on fait réagir l'halogénobenzène avec un acide sulfonique, un précurseur ou un dérivé de cet acide en présence de trifluorure de bore en quantité telle que la pression absolue de trifluorure de bore dans l'enceinte réactionnelle soit supérieure à 1 bar et en présence d'acide fluorhydrique comme solvant.

2. Procédé selon la revendication 1 caractérisé en ce que l'halogéno benzène a pour formule générale:

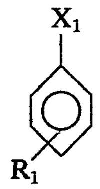

(I)

où:

- $X_1$ représente le chlore, le brome, l'iode ou le fluor
- et $R_1$ représente au moins un élément choisi parmi le groupe comprenant l'hydrogène et les radicaux alkyle et alkoxy ayant de 1 à 6 atomes de carbone, les radicaux phényle et phénoxy substitués par au moins un groupement plus désactivant que le groupement $X_1$, OH, Cl, Br, I et F.

3. Procédé selon l'une quelconque des revendications précédentes caractérisé en ce que l'acide sulfonique, le précurseur ou le dérivé de cet acide a pour formule générale:

$$R_2{-}SO_2X_2 \qquad (II)$$

dans laquelle:

$R_2$ représente un radical aliphatique ou aromatique
$X_2$ représente un halogène, OH, $OR_3$, $NH_2$, $NHR_4$, $NR_5R_6$ où $R_3$, $R_4$, $R_5$ et $R_6$ sont un radical aromatique ou aliphatique.

4. Procédé selon la revendication 3 caractérisé en ce que $R_2$ représente un radical alkyle, phényle, alkylphényle, phénylalkyle ou phényle comportant au moins un substituant halogène, $NO_2$, CN, $NH_2$, COOH, $CF_3$, $CCl_3$ ou $CBr_3$.

5. Procédé selon l'une quelconque des revendications précédentes caractérisé en ce que on utilise une quantité d'acide fluorhydrique telle que le rapport molaire de l'acide fluorhydrique à l'halogénobenzène est compris entre 5 et 50.

6. Procédé selon l'une quelconque des revendications précédentes caractérisé en ce que l'acide fluorhydrique mis en œuvre est de l'acide fluorhydrique anhydre.

7. Procédé selon l'une quelconque des revendications précédentes caractérisé en ce que l'halogénobenzène et l'acide sulfonique, son précurseur ou son dérivé sont utilisés en quantité sensiblement équimolaire.

8. Procédé selon l'une quelconque des revendications précédentes caractérisé en ce que on utilise une quantité de trifluorure de bore telle que la pression absolue de $BF_3$ dans l'enceinte réactionnelle soit comprise entre 6 et 20 bars.

9. Procédé selon l'une quelconque des revendications précédentes caractérisé en ce que on opère à une température comprise entre −20° C et 150° C.

## Patentansprüche

1. Verfahren zur Sulfonylierung von Halogenbenzolen, dadurch gekennzeichnet, daß man das Halogenbenzol mit einer Sulfonsäure, einem Vorläufer oder einem Derivat dieser Säure in Gegenwart von Bortrifluorid, das in einer derartigen Menge vorliegt, daß der Absolutdruck des Bortrifluorids im Reaktionsraum über 1 bar liegt, und in Gegenwart von Fluorwasserstoffsäure als Lösungsmittel umsetzt.

2. Verfahren nach Anspruch 1, dadurch gekennzeichnet, daß das Halogenbenzol die allgemeine Formel besitzt:

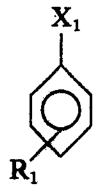

(I)

in der $X_1$ Chlor, Brom, Jod oder Fluor und $R_1$ wenigstens ein Element aus der Gruppe von Wasserstoff, den Alkyl- und Alkoxyresten mit 1 bis 6 Kohlenstoffatomen, den mit wenigstens einer stärker desaktivierenden Gruppe als die Gruppierung $X_1$, OH, Cl, Br, I und F substituierten Phenyl- und Phenoxyresten bedeuten.

3. Verfahren nach einem der vorangehenden Ansprüche, dadurch gekennzeichnet, daß die Sulfonsäure, der Vorläufer oder das Derivat dieser Säure die allgemeine Formel hat:

$$R_2-SO_2X_2 \qquad (II)$$

in der

$R_2$ einen aliphatischen oder aromatischen Rest bedeutet
$X_2$ ein Halogen, OH, $OR_3$, $NH_2$, $NHR_4$, $NR_5R_6$ bedeutet, wobei $R_3$, $R_4$, $R_5$ und $R_6$ einen aromatischen oder aliphatischen Rest darstellen.

4. Verfahren nach Anspruch 3, dadurch gekennzeichnet, daß $R_2$ ein Alkyl-, Phenyl-, Alkylphenyl-, Phenylalkyl- oder Phenylrest mit wenigstens einem der Substituenten Halogen, $NO_2$, CN, $NH_2$, COOH, $CF_3$, $CCl_3$ oder $CBr_3$ ist.

5. Verfahren nach einem der vorangehenden Ansprüche, dadurch gekennzeichnet, daß man eine derartige Menge an Fluorwasserstoffsäure einsetzt, daß das Molverhältnis zwischen Fluorwasserstoffsäure und dem Halogenbenzol zwischen 5 und 50 liegt.

6. Verfahren nach einem der vorangehenden Ansprüche, dadurch gekennzeichnet, daß die eingesetzte Fluorwasserstoffsäure wasserfrei ist.

7. Verfahren nach einem der vorangehenden Ansprüche, dadurch gekennzeichnet, daß das Halogenbenzol und die Sulfonsäure, ihr Vorläufer oder ihr Derivat in praktisch äquimolarer Menge eingesetzt werden.

8. Verfahren nach einem der vorangehenden Ansprüche, dadurch gekennzeichnet, daß man eine derartige Menge Bortrifluorid einsetzt, daß der Absolutdruck des $BF_3$ im Reaktionsraum zwischen 6 und 20 bar liegt.

9. Verfahren nach einem der vorangehenden Ansprüche, dadurch gekennzeichnet, daß man bei einer Temperatur zwischen —20° C und 150° C arbeitet.

**Claims**

1. Process for the sulphonylation of halogenobenzenes, characterised in that the halogenobenzene is reacted with a sulphonic acid or a precursor or derivative of this acid in the presence of boron trifluoride used in such an amount that the absolute pressure of boron trifluoride in the reaction space is greater than 1 bar, and in the presence of hydrofluoric acid as the solvent.

2. Process according to Claim 1, characterised in that the halogenobenzene has the general formula:

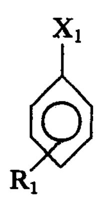

(I)

where $X_1$ represents chlorine, bromine, iodine or fluorine and $R_1$ represents at least one element chosen from the group comprising hydrogen, alkyl and alkoxy radicals having 1 to 6 carbon atoms, phenyl and phenoxy radicals substituted by at least one group which is more highly deactivating than the group $X_1$, OH, Cl, Br, I and F.

3. Process according to any one of the preceding claims, characterised in that the sulphonic acid, the precursor or the drivative of this acid has the general formula

$$R_2-SO_2X_2 \qquad (II)$$

in which $R_2$ represents an aliphatic or aromatic radical and $X_2$ represents a halogen, OH, $OR_3$, $NH_2$, $NHR_4$ or $NR_5R_6$, where $R_3$, $R_4$, $R_5$ and $R_6$ are an aromatic or aliphatic radical.

4. Process according to Claim 3, characterised in that $R_2$ represents an alkyl, phenyl, alkylphenyl, phenylalkyl or phenyl radical containing at least one halogen, $NO_2$, CN, $NH_2$, COOH, $CF_3$, $CCl_3$ or $CBr_3$ substituent.

5. Process according to any one of the preceding claims, characterised in that the amount of hydrofluoric acid used is such that the molar ratio of hydrofluoric acid to halogenobenzene is between 5 and 50.

6. Process according to any one of the preceding claims, characterised in that the hydrofluoric acid employed is anhydrous hydrofluoric acid.

7. Process according to any one of the preceding claims, characterised in that the halogenobenzene and the sulphonic acid, its precursor or its derivative are used in a substantially equimolar amount.

8. Process according to any one of the preceding claims, characterised in that the amount of boron trifluoride used is such that the absolute pressure of $BF_3$ in the reaction space is between 6 and 20 bars.

9. Process according to any one of the preceding claims, characterised in that it is carried out at a temperature of between −20° C and 150° C.